# EUROPÄISCHE PATENTANMELDUNG

(11) **EP 1 415 801 A2**
(43) Veröffentlichungstag der Anmeldung: **06.05.2004**
(21) Anmeldenummer: 03020239.4
(22) Anmeldetag: 06.09.2003
(51) Int. Cl.: B32B 25/14, A43B 17/00

(54) **Biegeelement mit drehsinnabhängigem Verhalten**

(30) Priorität: 29.10.2002 DE 10250330
(71) Anmelder: Degussa AG, 40474 Düsseldorf (DE)
(72) Erfinder: Monsheimer, Sylvia, 45721 Haltern am See (DE); Beyer, Michael, 46348 Raesfeld (DE)

(57) **Zusammenfassung**

Ein anisotropes Biegeelement, das mindestens folgende Schichten enthält:
I. Eine verstärkende Schicht I, die eine faserförmige verstärkende Komponente I mit einem Zug-E-Modul von 1 800 bis 20 000 N/mm² enthält, sowie
II. eine elastomere Schicht II mit einem Zug-E-Modul von 2 bis 1 300 N/mm²,
wobei die Komponente I der verstärkenden Schicht bzw. Schichten I und die Gesamtmenge an Elastomer der Schicht bzw. Schichten II zueinander im Gewichtsverhältnis 1 : 99 bis 40 : 60 stehen, und wobei bei Biegung um eine parallel zur verstärkenden Schicht I verlaufende Achse das Verhältnis der Steifigkeit des gesamten Biegeelements bei positivem Drehsinn zum Verhältnis der Steifigkeit bei negativem Drehsinn mindestens 1,2 beträgt, wird beispielsweise als Einlegeteil für Sportschuhe verwendet.

## Beschreibung

Die Erfindung betrifft anisotrope Formteile mit drehsinnabhängiger Steifigkeit bezüglich mindestens einer Raumrichtung. Die Formteile nehmen bei positivem Drehsinn deutlich höhere Kräfte auf als bei negativem Drehsinn und haben dadurch auch entsprechend höhere Rückstellkräfte. Die Erfindung betrifft weiterhin die Verwendung eines solchen Formteils als Einlegeteil für Sportschuhe.

Formteile mit anisotroper Steifigkeit sind bekannt. In der DE-OS 197 16 179 werden Formteile beschrieben, die sich bei Einwirkung von Kräften aus einer Richtung quer zur Längsrichtung steif und bei Einwirkung von Kräften aus anderen Richtungen deutlich weicher verhalten. Dies wird durch das Einbetten von Thermoplaststreifen in eine elastomere Matrix erreicht.

Der Erfindung liegt die Aufgabe zugrunde, ein Formteil herzustellen, das sich bei Biegung in mindestens einer Raumrichtung in positivem Drehsinn deutlich anders verhält als bei Biegung in negativem Drehsinn. So ist etwa bei Einlegeteilen für Fußballschuhe ein weiches Verhalten beim Laufen erforderlich, damit der Vorderfuß abrollen kann, wohingegen beim Schießen des Balles, also bei Umkehr des Drehsinns, eine deutliche Unterstützung des Fußes durch eine höhere Steifigkeit des Elementes von Vorteil ist.

Diese Aufgabe wird gelöst durch ein Formteil in Form eines anisotropen Biegeelements, das mindestens folgende Schichten enthält:
I. Eine verstärkende Schicht I, die eine faserförmige verstärkende Komponente I mit einem Zug-E-Modul gemäß DIN EN ISO 527 von 1 800 bis 20 000 N/mm² und vorzugsweise 12 000 bis 18 000 N/mm² enthält, sowie
II. eine elastomere Schicht II mit einem Zug-E-Modul gemäß DIN EN ISO 527 von 2 bis 1 300 N/mm², vorzugsweise 80 bis 800 N/mm²,
wobei die Komponente I der verstärkenden Schicht bzw. Schichten I und die Gesamtmenge an Elastomer der Schicht bzw. Schichten II zueinander im Gewichtsverhältnis 1 : 99 bis 40 : 60 stehen und wobei bei Biegung um eine in der Schicht liegende Achse das Verhältnis der Steifigkeit des gesamten Biegeelements bei positivem Drehsinn zum Verhältnis der Steifigkeit bei negativem Drehsinn mindestens 1,2 beträgt.

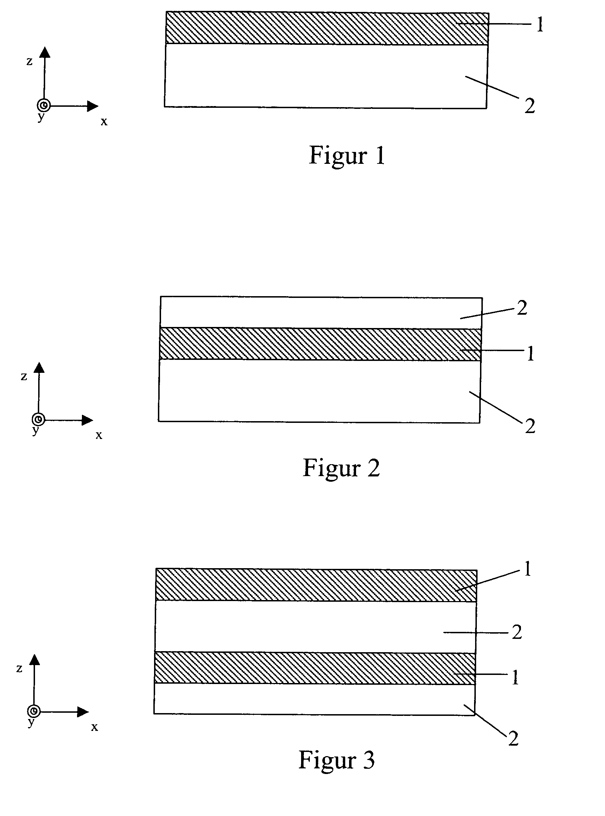

In einer Ausführungsform besteht das Formteil aus einer Schicht I **(1)** und einer Schicht II **(2)**, wie es in der **Figur 1** wiedergegeben ist. Die Abhängigkeit der Steifigkeit vom Drehsinn ist hier allerdings nur über die Profilhöhe bzw. Materialfestigkeit des weichen Teils (Schicht II) und/oder Art bzw. Menge der verstärkenden Komponente I einstellbar. Das Verhältnis der Steifigkeit je nach Drehsinn ist relativ hoch, die Steifigkeit in die weichere Richtung kann jedoch, da sie ausschließlich durch das weiche Material bestimmt wird, für manche Anwendungen zu gering sein.

In einer bevorzugten Ausführungsform besteht das Formteil aus zwei Schichten II **(2)**, zwischen denen eine Schicht I **(1)** außermittig angeordnet ist. Diese Anordnung ist in der **Figur 2** wiedergegeben. Die Abhängigkeit der Steifigkeit vom Drehsinn ist hier über die Lage der innenliegenden Verstärkungsschicht **(1)** einstellbar. Da aber die Schicht I bevorzugt Zugkräfte aufnimmt, trägt je nach Drehsinn nur eine der beiden Schichten II die Druckkräfte. Der Aufbau des Elementes ist daher relativ hoch.

In einer weiteren bevorzugten Ausführungsform besteht das Formteil aus zwei Schichten I **(1)** und zwei Schichten II **(2)**, wie es in der **Figur 3** wiedergegeben ist. Hier ist bei minimaler Bauhöhe die Abhängigkeit der Steifigkeit vom Drehsinn über die Lage der innenliegenden Verstärkungsschicht **(1)** einstellbar.

In Sonderfällen sind auch Ausführungsformen mit 5, 6, 7 oder mehr Schichten möglich, sie bedingen jedoch einen zunehmenden konstruktiven Aufwand.

Die erfindungsgemäßen Formteile sind im allgemeinen langgestreckte oder flächige Gebilde. Sie besitzen, wie in den Figuren 1, 2 und 3 gezeigt, zwei parallel zur verstärkenden Schicht I verlaufende Achsen x und y sowie eine senkrecht zur verstärkenden Schicht I verlaufende Achse z. Der Begriff "parallel" schließt hierbei ein, dass die Achsen x und y auch innerhalb einer der verstärkenden Schichten I liegen können. Ist das Biegeelement ein langgestrecktes Gebilde, so ist im allgemeinen die x-Achse die Längsachse und die y-Achse die Drehachse. Wenn das Biegeelement ein flächiges Gebilde ist, können sowohl die x-Achse als auch die y-Achse die Drehachse darstellen.

Die verstärkenden Fasern können jeden beliebigen Querschnitt besitzen, beispielsweise rund, oval oder abgeflacht. Ihr Durchmesser richtet sich nach den Dimensionen des Formteils; er liegt im allgemeinen im Bereich von 0,0001 mm bis 2 mm und bevorzugt im Bereich von 0,1 bis 0,6 mm. Die Verstärkungsfasern können aus den verschiedensten Materialien bestehen, z. B. aus Baumwolle, regenerierter Zellulose oder Acetylcellulose (Rayon), Polyethylenterephthalat, Polybutylenterephthalat, Polyethylen, Polypropylen, Polyamid, Aramid, Polyacrylnitril, Kohlenstoff, Bor, Stahl oder Glas. Sie können als Kurz-, Lang- oder Endlosfasern oder in Form von Rovings, Garnen, unidirektionalen Bändern (UD-Tapes), Vliesen, Geweben oder Gestricken und gegebenenfalls auch mit den verschiedensten Bindungsarten wie Leinwand-, Köper- oder Atlasbindung eingesetzt werden. Auch Mischgewebe aus verschiedenen Faserarten sowie Schichtstrukturen, die aus verschiedenen Vliesen und Geweben zusammengesetzt sind, lassen sich als Verstärkungskomponente verwenden.

Die elastomere Schicht II besteht aus einem Kautschuk, einem Gummi oder einem thermoplastischen Elastomer. Geeignete Kautschuke sind z. B. Styrol-Butadien-Kautschuke, Butadienkautschuk, Isoprenkautschuk, Naturkautschuk, Isobuten-Isopren-Kautschuk, Nitrilkautschuk, Chloroprenkautschuk, Ethylen/Propylen-Kautschuk (EPM), Ethylen/Propylen/Dien-Kautschuk (EPDM) oder Gemische hiervon. Diese Kautschuke bzw. daraus hergestellte Kautschukcompounds können im Formteil zu einem Gummi vulkanisiert sein. Alle diese Kautschuke sowie deren Vulkanisation sind dem Fachmann wohlbekannt, so dass sich eine nähere Beschreibung hier erübrigt. Die genaue Wahl des Kautschuks richtet sich nach den anwendungstechnischen Erfordernissen und ist im Rahmen der Erfindung nicht kritisch.

Ebenfalls geeignete thermoplastische Elastomere sind beispielsweise Polyetheresteramide, Polyetheramide, Polyetherester, Mischungen aus EPM bzw. EPDM und einem Polyolefin, Styrol/Butadien-Blockcopolymere, die gegebenenfalls auch hydriert sein können, thermoplastische Polyurethane, Mischungen hieraus oder Mischungen eines oder mehrerer dieser thermoplastischen Elastomere mit einem Kautschuk. Auch diese Stoffklassen sind dem Fachmann wohlbekannt.

Im Rahmen der Erfindung ist es wünschenswert, wenn das Elastomer der Schicht II und die verstärkende Komponente der Schicht I aufeinander haften. Gemäß dem Stand der Technik hat der Fachmann hier folgende Möglichkeiten:
- Wahl von Materialien, die als verträglich bekannt sind (z. B. PA12 als Verstärkung und Polyetheresteramid bzw. Polyetheramid auf PA12-Basis als Elastomer; PA11 als Verstärkung und Polyetheresteramid bzw. Polyetheramid auf PA11-Basis als Elastomer sowie entsprechende Kombinationen auf Basis anderer Polyamide wie z. B. PA6 oder PA612);
- Modifizierung einer Komponente, üblicherweise der Elastomerkomponente, durch Einpolymerisieren oder Aufpfropfen einer Verbindung mit einer reaktiven Gruppe, beispielsweise Maleinsäureanhydrid;
- Verwendung eines Haftvermittlers.

Die erfindungsgemäßen Formteile können nach verschiedenen Methoden hergestellt werden. Üblicherweise wird die Schicht I in einem gesonderten Arbeitsgang hergestellt. Es gibt eine Reihe von Methoden, die zu verstärkenden Elementen führen, in denen die Verstärkungsfasern vom Elastomeren gut benetzt und fest umschlossen sind. Von diesen Methoden seien beispielhaft die folgenden genannt:
a) Die Verstärkungsfasern in ihrer jeweiligen Anwendungsform (z. B. Rovings, Gewebe, Gewirke, UD-Tapes) werden mit einer Lösung des Elastomeren getränkt, das Lösungsmittel wird verdampft und gleichzeitig oder anschließend erhält das verstärkende Element seine Form, z. B. durch einen Pressvorgang bei erhöhter Temperatur.
b) Die Verstärkungsfasern in ihrer jeweiligen Anwendungsform und dünne Folien des thermoplastischen Elastomeren werden alternierend aufeinandergelegt, und der Schichtkörper wird unter Druck und bei erhöhter Temperatur in einem Autoklaven oder einer Presse verformt.
c) Auf die Verstärkungsfasern in ihrer jeweiligen Anwendungsform, vorteilhafterweise ein Vlies, Gewebe oder Gewirke, wird ein Pulver des thermoplastischen Elastomeren gestreut, worauf die Fasern mit dem anhaftenden Pulver durch eine Heizstrecke geführt werden, vorzugsweise durch einen Infrarotofen, wo das Polymerpulver schmilzt und an den Fasern festklebt. Das so entstehende Prepreg wird über einen Kalander abgekühlt und kann dann, z. B. in einer Presse bei erhöhter Temperatur, in die gewünschte Form gebracht werden.
d) Rovings können verarbeitet werden, indem sie zunächst gespreizt und in diesem Zustand im Wirbelbett mit dem Pulver aus dem Elastomer benetzt werden. Danach wird der benetzte Roving mit einem extrudierten Film aus demselben Polymeren überzogen. Diese Rovings können dann zu Gewebe- oder UD-Prepregs verarbeitet werden, die ihrerseits, z. B. in einer Presse unter Druck und bei erhöhter Temperatur, zum verstärkenden Element verformt werden können.

Diese und ähnliche Methoden haben also gemeinsam, dass in einem ersten Schritt die Verstärkungsschicht I als Prepreg hergestellt wird.

In einem zweiten Schritt wird das Prepreg mit der Elastomerschicht vereinigt. Dies kann beispielsweise dadurch geschehen, dass das Prepreg in eine Gieß- oder Spritzgießform eingelegt und mit der elastomeren Matrix überspritzt wird. Alternativ kann ein solches Formteil auch über Pressen hergestellt werden. Wenn es sich um einen Verbund aus nur zwei Schichten handelt, ist das so entstandene Verbundteil nun bereits fertig.

Falls das Verbundteil drei Schichten enthalten soll - außen elastomere Matrix, innen, aber nicht mittig, die Versteifungslage - so wird dieses überspritzte Teil wieder in eine Form eingelegt und von der anderen Seite ebenfalls überspritzt. Soll außen auch noch eine Versteifungslage sein (Vierschicht-Verbundteil), so wird diese als Prepreg ebenfalls eingelegt, und das Elastomere wird zwischen die beiden eingelegten Teile gespritzt.

Ist die verstärkende Komponente jedoch ausreichend grobfaserig, so kann sie auch ohne vorherige Verarbeitung zu einem Prepreg mit der oder den Elastomerschichten verpresst werden. Dabei dringt das Elastomer in die Zwischenräume zwischen den einzelnen Fasern ein und füllt sie aus.

Eine andere Möglichkeit, das erfindungsgemäße Biegeelement herzustellen, besteht bei der Verwendung von Kurzfasern als verstärkende Komponente darin, dass zur Herstellung der Schicht I ein kurzfaserverstärktes Elastomercompound verwendet wird und das Formteil mittels Zweikomponentenspritzguss hergestellt wird.

Das Verhältnis der Steifigkeit des gesamten Biegeelementes bei positivem Drehsinn zum Verhältnis der Steifigkeit bei negativem Drehsinn liegt bevorzugt zwischen 1 : 1,2 und 1 : 6, besonders bevorzugt zwischen 1 : 1,5 und 1 : 5 und ganz besonders bevorzugt zwischen 1 : 1,8 und 1 : 3.

Das Biegeelement kann über die Schichten I und II hinaus noch weitere Schichten enthalten, etwa eine Lackschicht, eine Klebschicht oder eine Schicht aus einer Dekorfolie. Es kann darüber hinaus weitere Konstruktionselemente enthalten, beispielsweise angespritzte Befestigungspunkte, etwa zur Krafteinleitung.

Das erfindungsgemäße Biegeelement wird für Anwendungen eingesetzt, bei denen es um eine parallel zur verstärkenden Schicht I verlaufende Achse sowohl in positivem als auch in negativem Drehsinn gebogen wird, beispielsweise als Einlegeteil für Sportschuhe, insbesondere Fußballschuhe, für Sportgeräte wie etwa Paddel oder in der Medizintechnik, etwa für Prothesen.

## Patentansprüche

1. Anisotropes Biegeelement, das mindestens folgende Schichten enthält:
I. Eine verstärkende Schicht I, die eine faserförmige verstärkende Komponente I mit einem Zug-E-Modul von 1 800 bis 20 000 N/mm² enthält, sowie
II. eine elastomere Schicht II mit einem Zug-E-Modul von 2 bis 1 300 N/mm²,
wobei die Komponente I der verstärkenden Schicht bzw. Schichten I und die Gesamtmenge an Elastomer der Schicht bzw. Schichten II zueinander im Gewichtsverhältnis 1 : 99 bis 40 : 60 stehen, und wobei bei Biegung um eine parallel zur verstärkenden Schicht I verlaufende Achse das Verhältnis der Steifigkeit des gesamten Biegeelements bei positivem Drehsinn zum Verhältnis der Steifigkeit bei negativem Drehsinn mindestens 1,2 beträgt.

2. Biegeelement gemäß Anspruch 1,
**dadurch gekennzeichnet,**
**dass** das Verhältnis der Steifigkeit des gesamten Biegeelements bei positivem Drehsinn zum Verhältnis der Steifigkeit bei negativem Drehsinn zwischen 1 : 1,2 und 1 : 6 liegt.

3. Biegeelement gemäß Anspruch 1,
**dadurch gekennzeichnet,**
**dass** das Verhältnis der Steifigkeit des gesamten Biegeelements bei positivem Drehsinn zum Verhältnis der Steifigkeit bei negativem Drehsinn zwischen 1 : 1,5 und 1 : 5 liegt.

4. Biegeelement gemäß Anspruch 1,
**dadurch gekennzeichnet,**
**dass** das Verhältnis der Steifigkeit des gesamten Biegeelements bei positivem Drehsinn zum Verhältnis der Steifigkeit bei negativem Drehsinn zwischen 1 : 1,8 und 1 : 3 liegt.

5. Biegeelement gemäß einem der Ansprüche 1 bis 4,
**dadurch gekennzeichnet,**
**dass** es eine einzige Schicht I und eine einzige Schicht II enthält.

6. Biegeelement gemäß einem der Ansprüche 1 bis 4,
**dadurch gekennzeichnet,**
**dass** es zwei Schichten II enthält, zwischen denen eine Schicht I außermittig angeordnet ist.

7. Biegeelement gemäß einem der Ansprüche 1 bis 4,
**dadurch gekennzeichnet,**
**dass** es die Schichtenanordnung
- Schicht I
- Schicht II
- Schicht I
- Schicht II
enthält.

8. Biegeelement gemäß einem der Ansprüche 1 bis 7,
**dadurch gekennzeichnet,**
**dass** es ein Einlegeteil für einen Sportschuh ist.

9. Biegeelement gemäß einem der Ansprüche 1 bis 7,
**dadurch gekennzeichnet,**
**dass** es Bestandteil eines Sportgerätes ist.

10. Biegeelement gemäß einem der Ansprüche 1 bis 7,
**dadurch gekennzeichnet,**
**dass** es für medizintechnische Anwendungen verwendet wird.

11. Verwendung eines Biegeelements gemäß einem der Ansprüche 1 bis 7 für Anwendungen, bei denen es um eine parallel zur verstärkenden Schicht I verlaufende Achse sowohl in positivem Drehsinn als auch in negativem Drehsinn gebogen wird.
